# EUROPEAN PATENT SPECIFICATION

(11) **EP 4 144 395 B1**
(45) Date of publication and mention of the grant of the patent: **06.11.2024**
(21) Application number: 21809685.7
(22) Date of filing: 09.04.2021
(51) Int. Cl.: A61M 25/00, A61M 25/14

(54) **QUADRUPLE-LUMEN CATHETER**
VIERFACHLUMENKATHETER
CATHÉTER À QUATRE LUMIÈRES

(30) Priority: 22.05.2020 JP 2020089928
(43) Date of publication of application: 08.03.2023
(73) Proprietor: Nipro Corporation, Osaka-shi, Osaka 531-8510 (JP)
(72) Inventor: MATSUDA, Yusuke, Osaka-shi, Osaka 531-8510 (JP); NAKAMURA, Takuma, Osaka-shi, Osaka 531-8510 (JP); FUJIKI, Yuya, Osaka-shi, Osaka 531-8510 (JP); NAKAI, Shota, Osaka-shi, Osaka 531-8510 (JP)
(74) Representative: Germain Maureau
(86) International application number: PCT/JP2021/015069
(87) International publication number: WO 2021/235135

(56) References cited:
- JP-A- 2008 000 441
- JP-A- 2011 255 179
- JP-A- 2016 022 192
- JP-A- 2018 198 930
- US-A1- 2013 261 605
- US-A1- 2017 273 628
- No further relevant documents disclosed

## Description

### TECHNICAL FIELD

The present disclosure relates to a quadruple-lumen catheter.

### BACKGROUND ART

For artificial dialysis, a catheter provided with a blood removal lumen which removes blood from the subject's body and a blood return lumen which returns the purified blood to the subject's body is used. Further, a quadruple-lumen catheter is known, which can handle cases where it is necessary to perform administration of drug solution, measurement of central venous pressure, or the like concurrently with the hemodialysis (e.g., see Patent Document 1). Such a quadruple-lumen catheter is further provided with two infusion lumens in addition to the blood removal lumen and the blood return lumen. This eliminates the need for preparing a new route for the administration of drug solution or the measurement of central venous pressure, thereby reducing the burden on an operator and the patient.

When blood is sucked through the blood removal lumen, there would be such a case that the inner wall of blood vessel stick to an opening of the blood removal lumen due to the suction pressure. This would cause such a drawback that the opening of the blood removal lumen is blocked, thereby causing blood removal failure or a decrease in removal blood flow rate. In some cases, the drawback would be solved by reverse connection in which the blood removal lumen and the blood return lumen are exchanged to return the blood through the blood removal lumen, thereby solving sticking of the inner wall of the blood vessel to the opening (e.g., see Patent Document 2).

US2013/261605A1 discloses a multi-lumen catheter including an elliptical cross-sectional profile configuration that enhances fluid flow rate while minimizing the average diameter of the catheter body. In one embodiment the catheter includes an elongate catheter tube defining a plurality of lumens. At least a portion of the longitudinal length of the catheter tube may define an elliptical cross section, in turn defined by a major axis and a minor axis. In one embodiment, a ratio of the major axis to the minor axis of the elliptical cross section is between about 1.3 and about 1.4. The catheter body can define two, three, or more lumens. In one embodiment, a dual-lumen catheter tube includes an aspect ratio of about 1.1:1, and each lumen thereof includes an inner surface defined by a plurality of radii and an hourglass-shaped septum.

JP 2016 022192 A discloses a catheter including: a blood return lumen which is arranged in a first region and communicates with the outside via an opening part provided in a distal end part; a blood removal lumen which is arranged in a second region and communicates with the outside via a semicircular opening part arranged on a proximal end part side with respect to the opening part of the blood return lumen; three or more transfusion lumens arranged in the first region; and a narrow diameter part which is provided from the opening part of the blood removal lumen to the distal end part, has the blood return lumen and three or more transfusion lumens in the inside, and has a taper so that a diameter is reduced gradually toward the distal end part. Then, a cross section of the blood return lumen is formed to be bigger than cross sections of the respective transfusion lumens. The opening part of the blood removal lumen is slanted to the proximal end part side in an axial direction. The respective opening parts of the transfusion lumens are arranged in the narrow diameter part and are formed to follow the taper of the narrow diameter part.

JP 2011 255179 A discloses a dialysis catheter including a first portion having an outer wall having a first diameter, an elongated distal portion having a second diameter smaller than the first diameter, and a transition region between the first portion and distal portion. A first longitudinally extending venous lumen is configured to deliver blood. First and second independent longitudinally extending arterial lumens are configured to withdraw blood from a patient. The venous lumen and arterial lumen have first and second regions each positioned a first distance from the outer wall of the catheter and a third region positioned a second distance, which is shorter than the first distance, from the outer wall of the catheter to form an arch-shaped wall portion progressively increasing in thickness from the third region toward the first region and from the third region to the second region.

US2017/273628A1 discloses a catheter assembly including an elongate catheter tube. The number of lumens defined by the catheter tube can vary as a function of longitudinal length along the catheter tube. For instance, the catheter tube can define three lumens from the proximal end of the catheter tube and terminate one of the lumens at an intermediate termination point such that only two lumens are defined further distally along the catheter tube. A sensor can be placed in the terminating lumen so as to isolate it from the other lumens and from blood or other body fluids while still enabling the sensor to reside within the patient body when the catheter tube is positioned in the patient for use. In addition to this, various other lumen transition and sensor configurations are disclosed.

### CITATION LIST

### PATENT DOCUMENTS

PATENT DOCUMENT 1: Japanese Unexamined Patent Publication No. 2008-441
PATENT DOCUMENT 2: Japanese Unexamined Patent Publication No. 2018-198930

### SUMMARY OF THE DISCLOSURE

### TECHNICAL PROBLEMS

The quadruple-lumen catheter disclosed in Patent Document 1 is provided with a blood return lumen in a distal end portion of the catheter. Thus, the quadruple-lumen catheter disclosed in Patent Document 1 has such a problem that such reverse connection for removing blood through the blood return lumen provided in the distal end portion will result in recirculation in which blood returning from the blood removal lumen provided more proximal than the blood return lumen reenters the blood return lumen in the distal end portion. The same is true in reverse connection for two infusion lumens for supplying drug solution, and such reverse connection also causes similar recirculation.

It is an object of the present disclosure to provide a quadruple-lumen catheter in which recirculation of blood and a drug solution is difficult to occur even when the blood is removed through the blood return lumen for a reason such as resolving the sticking to the inner wall of the blood vessel, or an erroneous connection.

### SOLUTIONS TO THE PROBLEMS

A quadruple-lumen catheter according to an aspect of the present disclosure A quadruple-lumen catheter, including: a cylindrical body extending from a proximal end to a distal end and being surrounded by an outer wall, the body having an inner space divided into a first region and a second region by a partition extending in a longitudinal direction, the first region being divided into a first lumen and a third lumen being smaller in cross-sectional area than the first lumen, the second region being divided into a second lumen and a fourth lumen being smaller in cross-sectional area than the second lumen, the third lumen and the fourth lumen facing each other with the partition interposed therebetween, the second lumen having a second opening plane at its distal end, and the first lumen having a first opening plane at its distal end, and the second opening plane being more distal than the first opening plane, the third lumen having a third opening plane at its distal end, and the third opening plane being more distal than the second opening plane, the fourth lumen having a fourth opening plane at its distal end, and the fourth opening plane being more distal than the third opening plane, and the partition being such that, between the distal end of the body and the second opening plane, a width of the partition is gradually narrowed toward the third lumen and the fourth lumen.

Such a configuration makes it difficult to cause recirculation of blood and a drug solution even when the blood is removed through the first lumen or the second lumen. Such a configuration further makes it easy to insert the catheter into a blood vessel, thereby making it difficult to damage the blood vessel. In such a configuration, the first lumen and the second lumen can be large in cross-sectional area. This can more effectively making it difficult that the blood vessel sticks to the first lumen or the second lumen even when blood is removed through the first lumen or the second lumen.

The quadruple-lumen catheter according to an aspect may be so configured that the first lumen and the second lumen are symmetrical with respect to the partition, and the third lumen and the fourth lumen are symmetrical with respect to the partition. Such a configuration makes it difficult to cause recirculation of blood and a drug solution. Further, each lumen is symmetrical with respect to the partition. This facilitates extrusion molding in manufacturing of the quadruple-lumen catheter.

The quadruple-lumen catheter according to an aspect may be configured such that the first opening plane and the second opening plane are identical in size. With such a configuration, even when the first lumen or the second lumen is used as a blood removal lumen, the amount of blood removed can be secured, and the suction pressure can be lowered to more effectively make it difficult that the inner wall of the blood vessel sticks to the first opening plane or the second opening plane.

The quadruple-lumen catheter according to an aspect may be so configured that the first opening plane, the second opening plane, and the third opening plane are inclined toward the proximal end of the body with respect to the partition, and the fourth opening plane is orthogonal to the partition. Such a configuration facilitates insertion of the quadruple-lumen catheter into a blood vessel, thereby making it difficult to damage the blood vessel.

The quadruple-lumen catheter according to an aspect may be configured such that a portion of the body between the distal end thereof and the second opening plane has a lower hardness than a portion of the body between the second opening plane and the proximal end. Such a configuration facilitates insertion of the quadruple-lumen catheter into a blood vessel, thereby making it further difficult to damage the blood vessel.

The quadruple-lumen catheter according to an aspect may include a plurality of branch pipes provided to the proximal end of the body, the plurality of branch pipes being connected respectively to the first lumen, the second lumen, the third lumen, and the fourth lumen and being each provided with a connector at a proximal end thereof. With such a configuration, hemodialysis can be performed by connecting between the body and a blood circuit and the like via the connectors.

The quadruple-lumen catheter according to an aspect may be configured such that the first lumen has a plurality of first lumen side pores in its distal end portion, and the second lumen has a plurality of second lumen side pores in its distal end portion. With such a configuration, even when the first lumen or the second lumen is used as a blood removal lumen, the suction pressure can be lowered to make it difficult that the inner wall of the blood vessel sticks to the first opening plane or the second opening plane. Further, the removal blood flow rate can be easily ensured.

The quadruple-lumen catheter according to an aspect may be such that the outer wall has, in a portion where the first lumen is provided, a first lumen slit notching a distal end of the outer wall and/or in a portion where the second lumen is provided, a second lumen slit notching the distal end. With such a configuration, even when the first lumen or the second lumen is used as a blood removal lumen, the suction pressure can be diffused to more effectively make it difficult that the inner wall of the blood vessel sticks to the first opening plane or the second opening plane. Further, the configuration allows to easily ensure the removal blood flow rate.

### ADVANTAGES OF THE INVENTION

The invention is defined by claim 1 and further defined in dependent claims 2-7.

According to the quadruple-lumen catheter of the present disclosure, recirculation is difficult even when blood is removed through the blood return lumen for a reason such as resolving the sticking to the inner wall of the blood vessel, or an erroneous connection.

### BRIEF DESCRIPTION OF THE DRAWINGS

FIG. 1 is a side view of an entire quadruple-lumen catheter according to one embodiment.
FIG. 2 is a perspective view of the entire quadruple-lumen catheter according to the embodiment.
FIG. 3 is a perspective view of the entire quadruple-lumen catheter according to the embodiment.
FIG. 4 is a right-side view of the quadruple-lumen catheter according to the embodiment.
FIG. 5 is a left-side view of the quadruple-lumen catheter according to the embodiment.
FIG. 6 is a top view of the quadruple-lumen catheter of the embodiment.
FIG. 7 is a bottom view of the quadruple-lumen catheter of the embodiment.
FIG. 8 is a view of the quadruple-lumen catheter of FIG. 7 viewed from its distal end.
FIG. 9 is a view of the quadruple-lumen catheter of FIG. 7 viewed from its proximal end.
FIG. 10 is a perspective view of the quadruple-lumen catheter according to the embodiment.
FIG. 11 is a side view of an entire quadruple-lumen catheter according to a variation.
FIG. 12 is a side view of the entire quadruple-lumen catheter according to the variation.
FIG. 13 is a right-side view of the quadruple-lumen catheter according to the variation.
FIG. 14 is a left-side view of the quadruple-lumen catheter according to the variation.
FIG. 15 is a top view of the quadruple-lumen catheter according to the variation.
FIG. 16 is a bottom view of the quadruple-lumen catheter according to the variation.
FIG. 17 is a view of the quadruple-lumen catheter according to the variation viewed from its distal end.

### DESCRIPTION OF EMBODIMENTS

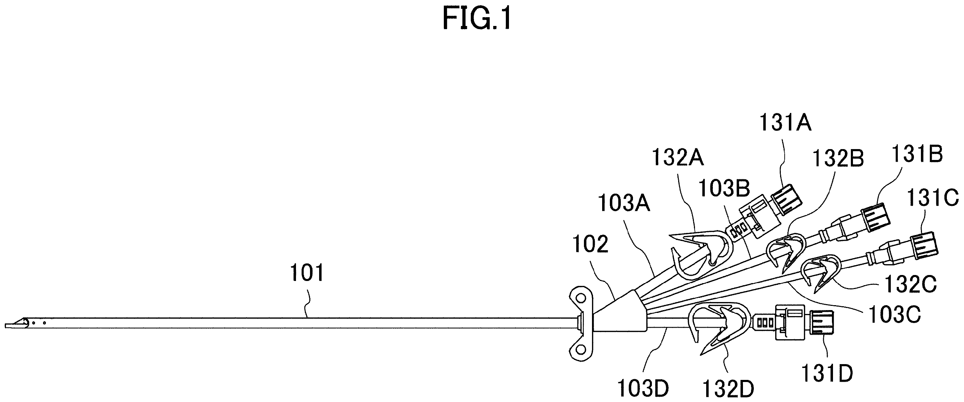

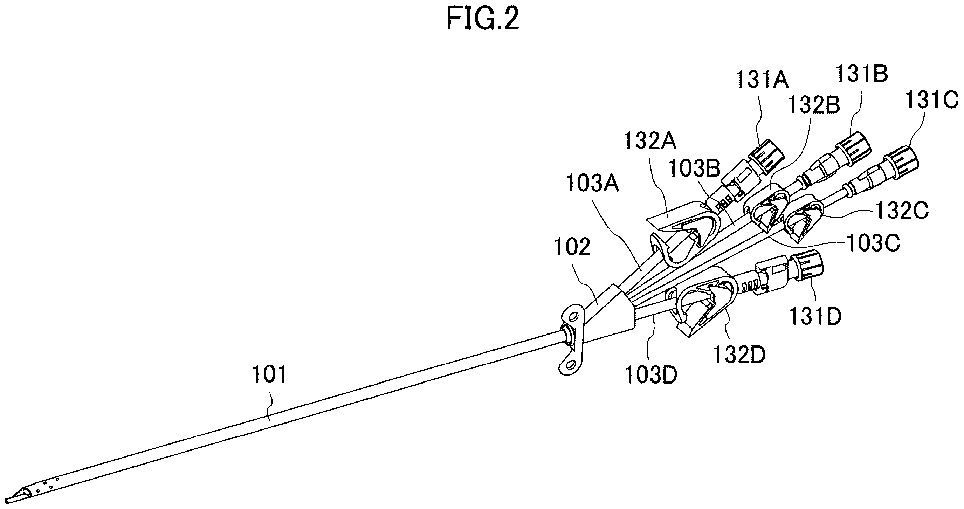

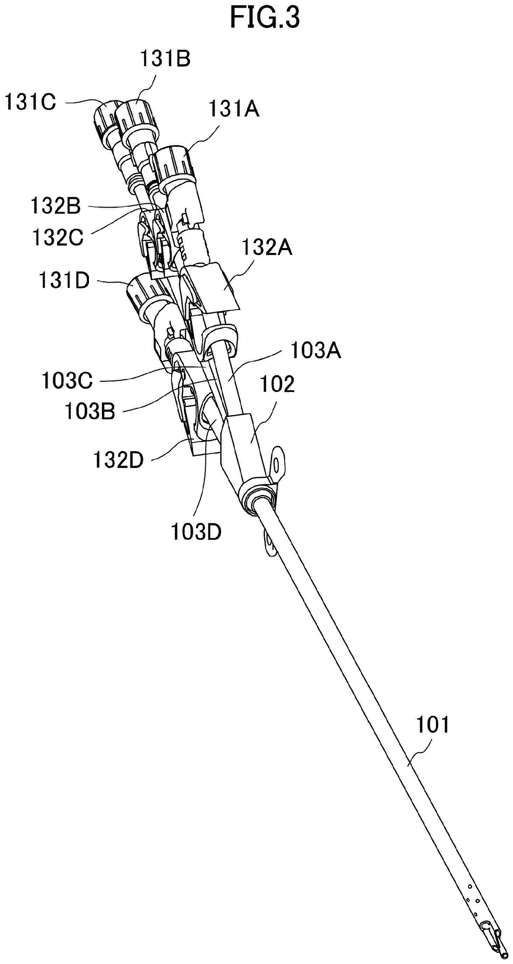

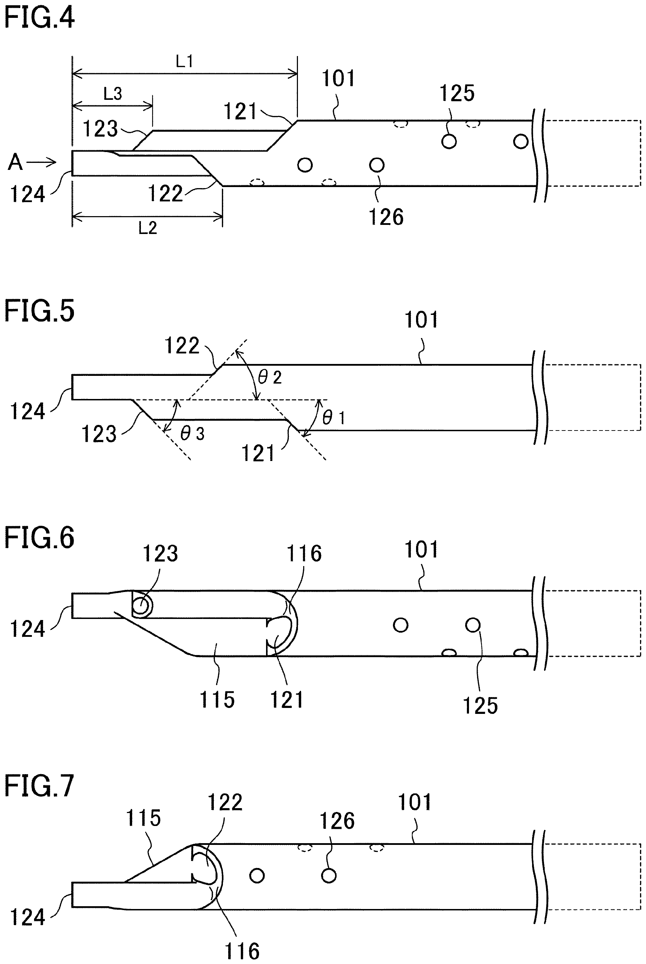

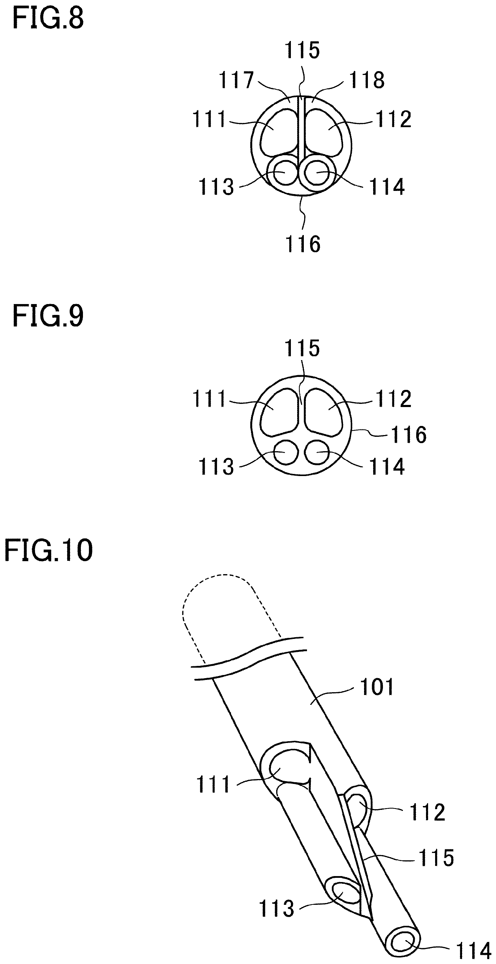

A quadruple-lumen catheter of the present disclosure is for use in performing administration of drug solution, measurement of central venous pressure, or the like concurrently when performing blood purification by hemodialysis using a blood removal lumen and a blood return lumen. As illustrated in FIGS. 1 to 10, the quadruple-lumen catheter of the present disclosure includes: a cylindrical body (a catheter tube) 101 surrounded by an outer wall 116. A partition 115 extending in a longitudinal direction is provided inside the body 101. The partition 115 divides an inner space of the body 101 into a first region 117 and a second region 118. The first region 117 is divided into a first lumen 111 and a third lumen 113, and the second region 118 is divided into a second lumen 112 and a fourth lumen 114. During normal forward connection, the first lumen 111 serves as a blood removal lumen, and the second lumen 112 serves as a blood return lumen. During a reverse connection in which the blood removal lumen and the blood return lumen are switched to solve sticking to the blood vessel wall, the first lumen 111 serves as a blood return lumen, and the second lumen 112 serves as a blood removal lumen. The third lumen 113 and the fourth lumen 114 serve as an infusion lumen to be used as routes for administration of drug solution, measurement of central venous pressure, or the like.

The third lumen 113 and the fourth lumen 114 are each circular in cross section, and the third lumen 113 and the fourth lumen 114, being on one of sides where the outer wall 116 and the partition 115 are in contact with each other, are positioned to face each other with the partition 115 interposed therebetween. The first lumen 111 and the second lumen 112 are positioned to face each other with the partition 115 interposed therebetween. In such a configuration, the third lumen 113, which serves as an infusion lumen, is provided in the first region 117, and the fourth lumen 114, which serves as the other infusion lumen, is provided in the second region 118. This allows to have the first lumen 111 and the second lumen 112 with larger cross-sectional areas, while two infusion lumens are provided. Therefore, even when either the first lumen 111 or the second lumen 112 is used as the blood removal lumen, the amount of blood removed can be secured and the suction pressure can be lowered to make the sticking of the blood vessel difficult. Further, the first lumen 111 and the second lumen 112, and the third lumen 113 and the fourth lumen 114 are symmetrically formed, in pair, with the partition 115 interposed therebetween. This facilitates extrusion molding for producing the quadruple-lumen catheter.

The distal ends of the first lumen 111, the second lumen 112, the third lumen 113, and the fourth lumen 114 are of an end hole-type with an opening at the distal end, thereby forming a first opening plane 121, a second opening plane 122, a third opening plane 123, and a fourth opening plane 124, respectively. At the first opening plane 121, the outer wall 116 is so trimmed that the outer wall 116 becomes farther from the partition 115 toward the proximal end. Thus, the first opening plane 121 is inclined toward the proximal end with respect to the partition 115. The second opening plane 122 is provided more distally than the first opening plane 121, and, at the second opening plane 122, the outer wall 116 is so trimmed that the outer wall 116 becomes farther from the partition 115 toward the proximal end. Thus, the second opening plane 122 is inclined toward the proximal end with respect to the partition 115. The third opening plane 123 is provided more distally than the second opening plane 122, and, at the third opening plane 123, the outer wall 116 is so trimmed that the outer wall 116 becomes farther from the partition 115 toward the proximal end. Thus, the third opening plane 123 is inclined toward the proximal end with respect to the partition 115. The fourth opening plane 124 is provided more distally than the third opening plane 123, and located at the distal end of the body 101, and the fourth opening plane 124 is orthogonal to the partition 115.

Although the use of the quadruple-lumen catheter of the present embodiment is not limited in terms of which direction is the upward, downward, rightward, or leftward direction of the quadruple-lumen catheter, the following description assumes that, with respect to the partition 115, the upper side is the side on which the first region 117 provided with the first lumen 111, and the lower side is the side on which a second region 118 provided with the third lumen 113 is located, and the right side is the side on which the first lumen 111 and the second lumen 112 are located, while the left side is the side on which the third lumen 113 and the fourth lumen 114 are located, when the body 101 is viewed from the distal end with the first region 117 on the upper side.

In the quadruple-lumen catheter of the present embodiment, the partition 115 is provided between the first lumen 111 and the second lumen 112, and the partition 115 extends toward the distal end beyond the first opening plane 121 of the first lumen 111. Thus, during the reverse connection, blood which returns through the first lumen 111 to flow above the partition 115 is less likely to be sucked via the second opening plane 122 below the partition 115, thereby making it difficult for the recirculation to occur. The making it difficult to cause the recirculation is advantageous not only in the case where the reverse connection is intentionally performed to resolve the sticking to the blood vessel wall, but also in the case of erroneous reverse connection.

Moreover, the partition 115 extends toward the distal end of the body 101 beyond the position of the second opening plane 122, and, from the position of the second opening plane 122 to the distal end, the partition 115 becomes narrower in width toward the left side on which the third lumen 123 and the fourth lumen 124 are disposed, and disappears at the distal end. This makes it easy to inset the catheter by the Seldinger technique or the like, and also makes the partition 115 less damaging to the blood vessel. In the present embodiment, the partition 115 has a width gradually narrowed toward the distal end from the position of the second opening plane 122. However, in view of preventing recirculation, the partition 115 has a width gradually narrowed toward the distal end from somewhere between the second opening plane 122 and the distal end.

For the blood removal via the first lumen 111, it is preferable that the first opening plane 121 be located as far away from the third opening plane 123 and the fourth opening plane 124 as possible for the sake of avoiding recirculation of the drug solution supplied through the third lumen 113 and the fourth lumen 114. In view of the length of the catheter, it is preferable that the distance from the distal end of the body 101 be not too long. More specifically, a distance L1 from the distal end (the fourth opening plane 124) of the body 101 to the first opening plane 121 is preferably 1 cm or longer, or more preferably 1.5 cm or longer, but preferably 10 cm or shorter, more preferably 5 cm or shorter, or yet more preferably 3 cm or shorter.

The first opening plane 121 is inclined toward the proximal end with respect to the partition 115. The inclination of the first opening plane 121 toward the proximal end can reduce insertion resistance in intracorporeally inserting the catheter by the Seldinger technique or the like, whereby the insertion of the catheter can be smooth, as compared with the case where the first opening plane 121 is orthogonal to the partition 115. Further, the inclination also makes the first opening plane 121 less damaging to the blood vessel. Further, the inclination gives the first opening plane 121 a larger size (a larger opening plane area) than that in the case where the first opening plane 121 is orthogonal to the partition 115. This makes it easier to ensure the blood flow rate and lower of the suction pressure in removing the blood through the first lumen 111.

To be less damaging to the blood vessel, the first opening plane 121 has an inclination angle θ1 that may be preferably 60° or less, more preferably 50° or less, or yet more preferably 40° or less. On the other hand, for performing the blood removal through the first lumen 111 in such a way that the wall of the blood vessel is prevented from sticking to the first opening plane 121 due to the suction pressure caused by the blood removal and consequently closing the first opening plane 121, the inclination angle θ1 may be preferably 10° or more, or more preferably 15° or more.

It is preferable that the second opening plane 122 be located as far away from the first opening plane 121 as possible in light of preventing recirculation between the first lumen 111 and the second lumen 112. On the other hand, it is preferable that the second lumen 112 be located as far away from also the third opening plane 123 as possible in light of preventing recirculation between the second lumen 112 used as a blood removal lumen during reverse connection and the third lumen 113. Thus, a distance L1 - L2 between the first opening plane 121 and the second opening plane 122 may be preferably 10% or more, more preferably 20% or more, and preferably 60% or less, or more preferably 50% or less of the distance L1 between the first opening plane 121 and the fourth opening plane 124.

Similarly to the first opening plane 121, the second opening plane 122 is inclined toward the proximal end with respect to the partition 115. This reduces the insertion resistance in intracorporeally inserting the catheter by the Seldinger technique or the like, whereby the insertion of the catheter can be smooth, as compared with the case where the second opening plane 122 is orthogonal to the partition 115. Further, the inclination makes the second opening plane 122 less damaging to the blood vessel. Further, the inclination gives the second opening plane 122 a larger size (a larger opening plane area) than that in the case where the second opening plane 122 is orthogonal to the partition 115. This makes it easier to ensure the blood flow rate in removing the blood through the second lumen 112, and allows lowering the suction pressure. The second opening plane 122 and the first opening plane 121 may be identical in size in consideration of using the second lumen 112 as a blood removal lumen.

An inclination angle θ2 of the second opening plane 122 may be preferably 60° or less, more preferably 50° or less, or yet more preferably 40° or less. The inclination angle θ2 may be preferably 10° or more, or more preferably 15° or more in light of making it possible to remove blood through the second lumen 112.

It is preferable that the third opening plane 123 be located as far away from the first opening plane 121 and the second opening plane 122 as possible in light of preventing recirculation between the first lumen 111 serving as a blood removal lumen during forward connection and the third lumen 113 and between the second lumen 112 serving as a blood removal lumen during reverse connection and the third lumen 113. Thus, a distance L1 - L3 between the first opening plane 121 and the third opening plane 123 may be preferably 30% or more, or more preferably 40% or more, but preferably 70% or less, or more preferably 60% or less of the distance L1 between the first opening plane 121 and the fourth opening plane 124. Moreover, a distance L2 - L3 between the second opening plane 122 and the third opening plane 123 may be preferably 10% or more, or more preferably 25% or more, but preferably 70% or less, or more preferably 60% or less of the distance L2 between the second opening plane 122 and the fourth opening plane 124.

Similarly to the first opening plane 121 and the second opening plane 122, the third opening plane 123 is inclined toward the proximal end with respect to the partition 115. This reduces the insertion resistance in intracorporeally inserting the catheter into the subject's body by the Seldinger technique or the like, whereby the insertion of the catheter can smooth, as compared with the case where the third opening plane 123 is orthogonal to the partition 115. Further, the inclination makes third opening plane 123 less damaging to the blood vessel. The inclination angle θ3 of the third opening plane 123 may be preferably 70° or less, more preferably 60° or less, or yet more preferably 50° or less. The inclination angle θ1 of the first opening plane 121, the inclination angle θ2 of the second opening plane 122, and the inclination angle θ3 of the third opening plane 123 may be identical to or different from one another.

The fourth lumen 114 has a circular cross-sectional outer shape at the distal end of the body 101. This makes the fourth lumen 114 less damaging to the blood vessel, as compared with the case where the fourth lumen 114 has an angular cross-sectional outer shape. The fourth lumen 114 has such an outer diameter between the distal end and the third opening plane 123 that may be preferably 1 mm or greater, or more preferably 1.5 mm or greater, but preferably 4 mm or smaller, or more preferably 3 mm or smaller in light of the ease insertion of the catheter.

In the quadruple-lumen catheter of the present embodiment, the third opening plane 123 and the fourth opening plane 124 are located more distally than the second opening plane 122. Accordingly, when the second lumen 112 serves as a blood return lumen during the forward connection, a drug solution supplied through the third lumen 113 and the fourth lumen 114 is carried by the blood flow through the blood vessel and blood flowing out of the second opening plane 122, and flows away from the first opening plane 121 through which blood is removed. This makes it difficult to cause recirculation of the drug solution. On the other hand, even when the second lumen 112 in the second region 118 serves as a blood removal lumen during the reverse connection, the location of the third opening plane 123 being in the first region 117 which is on the opposite side to the second region 118 across the partition 115 results in that a drug solution supplied through the third lumen 113 is carried by the blood flow flowing out of the first opening plane 121, and flows away from the second opening plane 122 through which blood is removed. This makes it difficult to cause recirculation of the drug solution. Further, the fourth opening plane 124 is located at the distal end of the body 101. Thus, even when the second lumen 112 serves as a blood removal lumen during the reverse connection, the drug solution supplied through the fourth lumen 114 is carried by the blood flow through the blood vessel and the blood flow flowing out of the first opening plane 121 and through the first region 117, and flows away from the second opening plane 122 through which blood is removed. This makes it difficult to cause recirculation of the drug solution.

In the quadruple-lumen catheter according to the present embodiment, the third lumen 113 and the fourth lumen 114 are provided in the vicinity of a portion where the outer wall 116 of the body 101 and the partition 115 are in contact with each other, in such a way that the third lumen 113 and the fourth lumen 114 face each other with the partition 115 interposed therebetween, and are disposed on one side (the left side) of the body 101. This makes it possible to provide the third opening plane 123 at a position more distal than the second opening plane 122, while gradually narrowing the width of a portion of the partition 115 from the second opening plane 122 toward the distal end. Thus, the quadruple-lumen catheter can be easily inserted into the blood vessel. The second opening plane 122 and the third opening plane 123 are arranged on opposite sides with respect to the central axis of the body 101, thereby being distanced from each other. Thus, recirculation is less likely to occur even in reverse connection. If the third lumen 113 and the fourth lumen 114 are arranged opposite sides with respect to the central axis of the body 101, it is necessary to separate the second opening plane 122 and the third opening plane 123 from each other by the partition 115 in order to prevent recirculation during reverse connection. Thus, the third opening plane 123 cannot be provided closer to the distal end than the second opening plane 122, while gradually narrowing the width of a portion of the partition 115 from the second opening plane 122 toward the distal end.

When blood is removed through the first lumen 111 and returns through the second lumen 112, the cross-sectional area of the first lumen 111 is preferably as large as possible to easily ensure the blood flow rate. However, in light of making it possible to remove blood also through the second lumen 112, the ratio of the cross-sectional area of the second lumen 112 to the cross-sectional area of the first lumen 111 may be preferably 0.6 or more, or more preferably 0.7 or more. The ratio of the cross-sectional area of the second lumen 112 to the cross-sectional area of the first lumen 111 may be 1, but may be preferably 0.95 or less, or more preferably 0.9 or less in light of ensuring the rate of blood removed through the first lumen 111. The cross-sectional area of the first region 117 and the cross-sectional area of the second region 118 into which the inside of the body 101 is divided by the partition 115 may be identical with each other, but the cross-sectional area of the first region 117 may be larger than the cross-sectional area of the second region 118 in light of increasing the cross-sectional area of the first lumen 111.

The cross-sectional areas of the first lumen 111 and the second lumen 112 and the sizes (the areas of the opening plane) of the first opening plane 121 and the second opening plane 122 may be such that the cross-sectional areas of the first lumen 111 and the second lumen 112 differ from each other, while the sizes of the first opening plane 121 and the second opening plane 122 are identical with each other. As an alternative, these may be so configured that the cross-sectional areas of the first lumen 111 and the second lumen 112 are identical with each other, while the sizes of the first opening plane 121 and the second opening plane 122 are identical with each other.

It is preferable that the first lumen 111 have such a cross-sectional area that is as large as possible in a portion of the first region 117 where the third lumen 113 is not present. Thus, the first lumen 111 may have a cross section other than a circular cross section. For example, the cross-sectional shape of the first lumen 111 may be a substantially circular shape such as an elliptical shape, a sector shape, or shapes distorted therefrom, in accordance with the shape of the portion of the first region 117 where the third lumen 113 is not present. Similarly, it is preferable that the second lumen 112 has such a cross-sectional area that is as large as possible in a portion of the second region 118 where the fourth lumen 114 is not present. Therefore, the second lumen 112 may have a substantially circular cross section in accordance with the shape of the portion of the second region 118 where the fourth lumen 114 is not present. The configuration in which the first lumen 111 and the second lumen 112 each are substantially circular in cross section can provide efficient utilization of the lumen of the body 101, and allows both the first lumen 111 and the second lumen 112 to be large in cross-sectional area. At least either one of the first lumen 111 or the second lumen 112 may have a circular cross section.

On the other hands, because the third lumen 113 and the fourth lumen 114 which each serve as an infusion lumen do not require a large flow rate, the cross-sectional areas of the third lumen 113 and the fourth lumen 114 are smaller than the cross-sectional areas of the first lumen 111 and the second lumen 112, and may be preferably 0.1 or more, more preferably 0.25 or more, and preferably 0.5 or less, more preferably 0.35 or less. The cross-sectional areas of the third lumen 113 and the fourth lumen 114 may be identical from each other, but the ratio of the cross-sectional area of the third lumen 113 to the cross-sectional area of the fourth lumen 114 may be changed within the range of about 0.5 to about 1.5 according to the intended use of each lumen.

The configuration in which the third lumen 113 and the fourth lumen 114 are each circular in cross section can reduce the resistance of fluid, thereby facilitating pumping, and also makes it easy to form the third lumen 113 and the fourth lumen 114. However, the third lumen 113 and the fourth lumen 114 may not be circular in cross section, and may be substantially circular in cross section.

The distal end portion of the first lumen 111 may be provided with a first lumen side pore 125 or first lumen side pores 125 communicating between the first lumen 111 and the outside. This configuration can reduce the suction pressure at the first opening plane 121 when the first lumen 111 serves as a blood removal lumen, thereby preventing the inner wall of the blood vessel from sticking to the first opening plane 121. Further, this configuration makes is possible to easily ensure the removal blood flow rate.

It is preferable to provide a plurality of the first lumen side pores 125 in order to attain a greater effect of dispersing the suction pressure. The number of first lumen side pores 125 is not particularly limited, but it is preferable to provide two first lumen side pores 125 each on an upper side and a right side of the first lumen 111. However, the first lumen side pores 125 may be provided as needed and may be omitted.

The distal end portion of the second lumen 112 may be provided with a second lumen side pore 126 or second lumen side pores 126 communicating between the second lumen 112 and the outside. This configuration can reduce the suction pressure at the second opening plane 122 when the second lumen 112 serves as a blood removal lumen, thereby preventing the inner wall of the blood vessel from sticking to the second opening plane 122. Further, this configuration makes it possible to easily ensure the removal blood flow rate.

It is preferable to provide a plurality of the second lumen side pores 126 in order to attain a greater effect of dispersing the suction pressure. The number of second lumen side pores 126 is not particularly limited, but it is preferable to provide two second lumens 112 each on an upper side and a right side of the second lumen 112. However, the second lumen side pores 126 may be provided as needed and may be omitted.

The length of the body 101 is not particularly limited, but may be preferably 10 cm or longer, or more preferably 13 cm or longer, but preferably 40 cm or shorter, or more preferably 30 cm or shorter.

The outer diameter of the body 101 is not particularly limited, but may be preferably 3 mm or greater, or more preferably 4 mm or greater in light of ensuring the blood flow rate. Moreover, for being insertable into the blood vessel, the outer diameter of the body 101 may be preferably 6 mm or smaller, or more preferably 5 mm or smaller.

The thickness of the outer wall 116 of the body 101 is not particularly limited, but is preferably 0.1 mm or more, more preferably 0.2 mm or more, and preferably 0.8 mm or less, more preferably 0.6 mm or less in light of the strength and flexibility of the catheter.

The body 101 may be formed of a material which is hard enough to maintain a stable shape in the blood vessel and but soft enough not to damage the blood vessel, such as polyurethane, polyvinyl chloride, silicone, polyethylene, polypropylene, an ethylene-vinyl acetate copolymer, or a polyamide. The material of the body 101 is particularly preferably polyurethane, which maintains such a hardness that does not impair the ease of insertion of the catheter and becomes harder at normal temperatures and softer at internal body temperatures. A distal end portion of the body 101, e.g., a portion of the body 101 between the distal end and the second opening plane 122 may be configured to be less damaging to blood vessels by being softer than the rest of the body 101. At least a portion of the body 101 may be formed of a material containing a contrast agent such as barium sulfate, bismuth tungstate, or bismuth oxide, so as to facilitate finding out where the body 101 is inserted. For example, by configuring such that the distal end of the body 101 has a color different from the color of the other portion of the body 101, the position of the distal end portion of the body 101 can be easily recognizable.

The body 101 is provided with branch pipes 103A to 103D extending from the proximal end of the body 101 via a branch portion 102. Distal ends of the branch pipes 103A to 103D are connected to the first lumen 111 to the fourth lumen 114, respectively. Each of the proximal ends of the branch pipes 103A to 103D is provided with a connector (not shown) connected thereto. The connectors, which are covered respectively with protection caps 131A to 131D in the illustrations in FIGS. 1 to 3, allow the body 101 to be connected to a blood circuit and the like via the connectors. The branch pipes 103A to 103D are provided respectively with clamps 132A to 132D attached thereto. With the clamps 132A to 132D, the branch pipes 103A to 103D can be closable for performing a treatment such as heparin rock.

The quadruple-lumen catheter of the present embodiment may be biocompatible-treated on at least one of an inner surface or an outer surface thereof with a biocompatible treatment of various types. For example, the quadruple-lumen catheter of the present embodiment may be antithrombotic-surfaced so as to prevent thrombus formation due to contact with blood during indwelling. The antithrombotic surfacing is not particularly limited and may be carried out by any methods such as a method of immobilizing a plasminogen activator such as urokinase or a method of immobilizing an anticoagulant such as heparin.

The catheter according to the present embodiment can be modified in various ways. In the following variations, only portions different from the present embodiment will be described. The description of the configuration common to the present embodiment will be omitted as appropriate. In addition, like portions are denoted with like reference numerals as in the present embodiment.

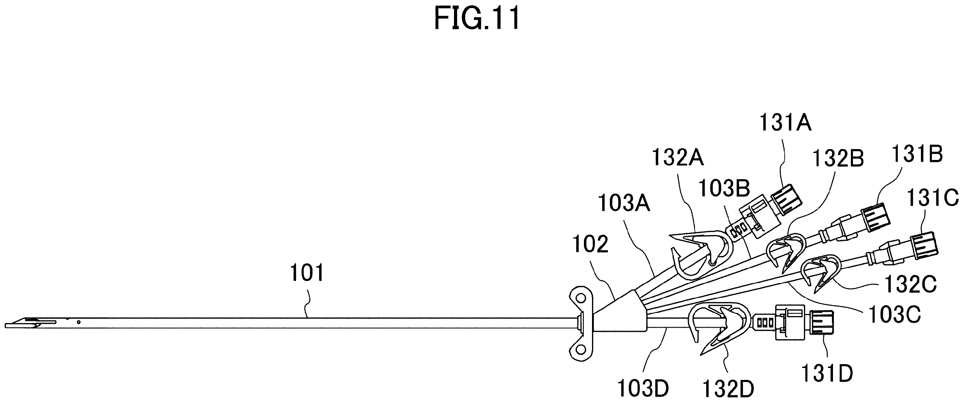

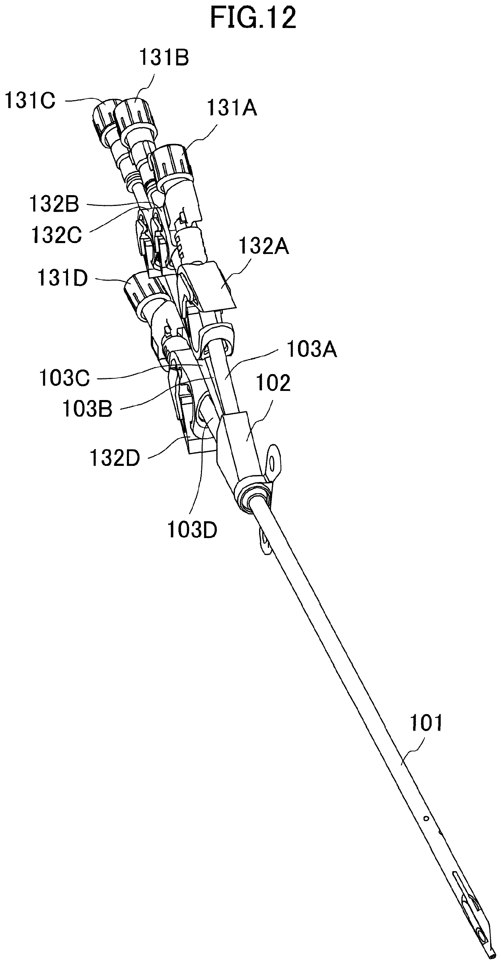

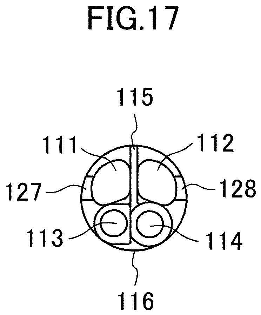

As a variation of the catheter according to the present embodiment, as shown in FIGS. 11 to 17, a first lumen slit 127 notching a portion of the distal end may be provided in a portion of the outer wall 116 where the first lumen 111 is provided. Further, a second lumen slit 128 notching a portion of the distal end may be provided in a portion of the outer wall 116 where the second lumen 112 is provided.

With such a configuration, the flow of blood sucked into the first lumen 111 is dispersed also into the first lumen slit 127 during forward connection. This reduces the suction pressure, thereby making it difficult to cause sticking to the blood vessel wall. With this configuration, even if the first opening plane 121 is clogged due to thrombus or the like, the first lumen 111 can be kept open. Further, with the second lumen slit 128, the same effect as in the configuration with the first lumen slit 127 can be obtained during the reverse connection.

Moreover, the body 101 may be so configured that the distal end portion of the body 101, e.g., a portion of the outer wall 116 between the distal end and a proximal end of the first lumen slit 127 is softer than the rest of the body 101 excluding the distal end portion. The configuration in which the first lumen slit 127 and the second lumen slit 128 notching portions of the distal end of the outer wall 116 are provided makes the outer wall 116 more easily deformable, but the configuration in which the distal end portion of the outer wall 116 is softer makes the outer wall 116 even more easily deformable. These configurations bring about such an effect that, when the catheter is inserted, the outer walls 116 forming the first lumen 111 and the second lumen 112 are easily deformed by bending inwardly toward the inside of the first lumen 111 and the inside of the second lumen 112. This provides such an excellent insertability to the catheter that, at inserting the catheter, the cross-sectional areas of the body 101 at the first opening plane 121 and the second opening plane 122 become smaller, thereby avoiding a sudden increase in insertion resistance.

The length L4 of the first lumen slit 127 and the length L5 of the second lumen slit 128 are not particularly limited, but may be preferably 2 mm or more but 15 mm or less, or more preferably 5 mm or more but 10 mm or less in light of reducing sticking of the catheter to the blood vessel wall. The length L4 of the first lumen slit 127 and the length L5 of the second lumen slit 128 may be identical to or different from each other.

It is sufficient that the widths of the first lumen slit 127 and the second lumen slit 128 are as predetermined. The widths of the first lumen slit 127 and the second lumen slit 128 are not particularly limited, and may be preferably 0.1 mm or more but 2.5 mm or less, or more preferably 0.5 mm or more but 2.0 mm or less in light of ensuring rigidity of the outer wall 116. The widths of the first lumen slit 127 and the second lumen slit 128 may be identical to or different from each other.

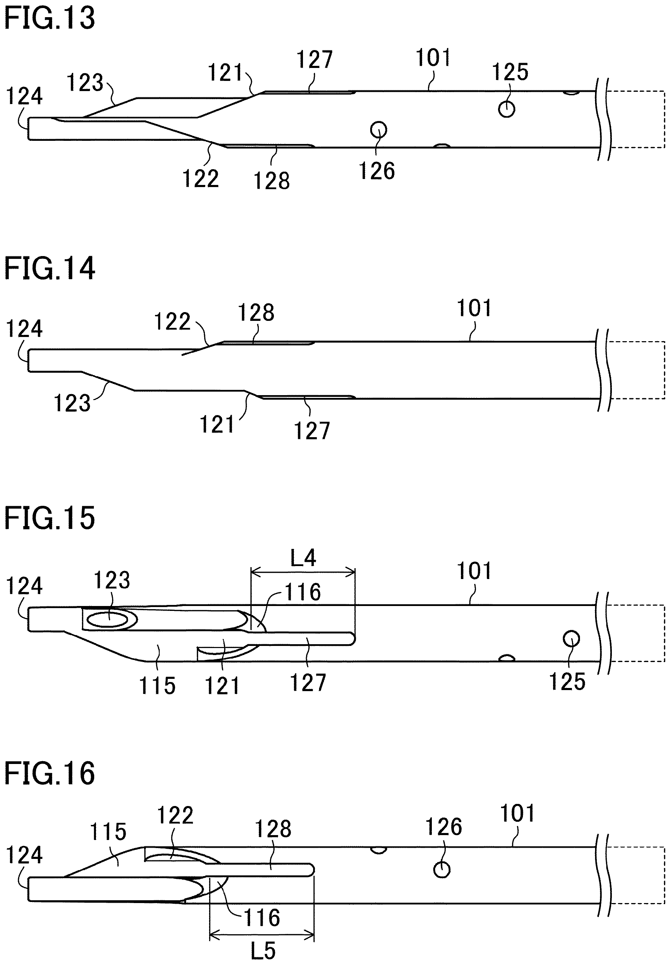

The first lumen slit 127 and the second lumen slit 128 illustrated in FIGS. 15 and 16 each are constant in width in the longitudinal direction, but the width is not limited to this, and the width may change in the longitudinal direction. For example, the first lumen slit 127 or the second lumen slit 128 may become wider gradually from its distal end to its proximal end.

Although the above discusses a variation in which the quadruple-lumen catheter is configured with the first lumen slit 127 and the second lumen slit 128, but the quadruple-lumen catheter may be configured with either the first lumen slit 127 or the second lumen slit 128.

### INDUSTRIAL APPLICABILITY

A quadruple-lumen catheter of the present disclosure makes it difficult to cause recirculation of blood and a drug solution even when blood is removed through the blood return lumen due to resolution of sticking to the inner wall of the blood vessel, an erroneous connection, or the like.

### DESCRIPTION OF REFERENCE CHARACTERS

- 101: Body
- 102: Branch Portion
- 103A: Branch Pipe
- 103B: Branch Pipe
- 103C: Branch Pipe
- 111: First Lumen
- 112: Second Lumen
- 113: Third Lumen
- 114: Fourth Lumen
- 115: Partition
- 116: Outer Wall
- 117: First Region
- 118: Second Region
- 121: First opening plane
- 122: Second opening plane
- 123: Third opening plane
- 124: Fourth opening plane
- 125: First Lumen Side Pore
- 126: Second Lumen Side Pore
- 127: First Lumen Slit
- 128: Second Lumen Slit
- 131A: Protective Cap
- 131B: Protective Cp
- 131C: Protective Cp
- 131D: Protective Cp
- 132A: Clamp
- 132B: Clamp
- 132C: Clamp
- 132D: Clamp

## Claims

1. A quadruple-lumen catheter, comprising:
a cylindrical body (101) extending from a proximal end to a distal end and being surrounded by an outer wall (116),
the body (101) having an inner space divided into a first region (117) and a second region (118) by a partition (115) extending in a longitudinal direction,
the first region (117) being divided into a first lumen (111) and a third lumen (113) being smaller in cross-sectional area than the first lumen (111),
the second region (118) being divided into a second lumen (112) and a fourth lumen (114) being smaller in cross-sectional area than the second lumen (112), **characterized in that**
the third lumen (113) and the fourth lumen (114) face each other with the partition (115) interposed therebetween,
the second lumen (112) has a second opening plane (122) at its distal end, and the first lumen (111) has a first opening plane (121) at its distal end, the second opening plane (122) being more distal than the first opening plane (121),
the third lumen (113) has a third opening plane (123) at its distal end, the third opening plane (123) being more distal than the second opening plane (122),
the fourth lumen (114) has a fourth opening plane (124) at its distal end, the fourth opening plane (124) being more distal than the third opening plane (123), and
the partition (115) is such that, between the distal end of the body (101) and the second opening plane (122), a width of the partition (115) is gradually narrowed toward the third lumen (113) and the fourth lumen (114).

2. The quadruple-lumen catheter of claim 1, wherein
the first lumen (111) and the second lumen (112) are symmetrical with respect to the partition (115), and the third lumen (113) and the fourth lumen (114) are symmetrical with respect to the partition (115).

3. The quadruple-lumen catheter of claim 1 or 2, wherein
the first opening plane (121) and the second opening plane (122) are identical in size.

4. The quadruple-lumen catheter of any one of claims 1 to 3, wherein
the first opening plane (121), the second opening plane (122), and the third opening plane (123) are inclined toward the proximal end of the body (101) with respect to the partition (115), and
the fourth opening plane (124) is orthogonal to the partition (115).

5. The quadruple-lumen catheter of any one of claims 1 to 4, wherein
a portion of the body (101) between the distal end thereof and the second opening plane (122) has a lower hardness than a portion of the body (101) between the second opening plane (122) and the proximal end.

6. The quadruple-lumen catheter of any one of claims 1 to 5, comprising:
a plurality of branch pipes (103A, 103B, 103C, 103D) provided to the proximal end of the body (101), the plurality of branch pipes (103A, 103B, 103C, 103D) being connected respectively to the first lumen (111), the second lumen (112), the third lumen (113), and the fourth lumen (114) and being each provided with a connector at a proximal end thereof.

7. The quadruple-lumen catheter of any one of claims 1 to 6, wherein
the first lumen (111) has a plurality of first lumen side pores (125) in its distal end portion, and
the second lumen (112) has a plurality of second lumen side pores (126) in its distal end portion.

8. The quadruple-lumen catheter of any one of claims 1 to 7, wherein
the outer wall (116) has, in a portion where the first lumen (111) is provided, a first lumen slit (127) notching a distal end of the outer wall (116) and/or in a portion where the second lumen (112) is provided, a second lumen slit (128) notching the distal end.

## Patentansprüche

1. Vierfachlumen-Katheter, umfassend:
einen zylindrischen Körper (101), der sich von einem proximalen Ende zu einem distalen Ende erstreckt und von einer Außenwand (116) umgeben ist,
wobei der Körper (101) einen Innenraum aufweist, der durch eine Trennwand (115), die sich in Längsrichtung erstreckt, in einen ersten Bereich (117) und einen zweiten Bereich (118) unterteilt ist,
wobei der erste Bereich (117) in ein erstes Lumen (111) und ein drittes Lumen (113) unterteilt ist, das eine kleinere Querschnittsfläche als das erste Lumen (111) aufweist,
wobei der zweite Bereich (118) in ein zweites Lumen (112) und ein viertes Lumen (114) unterteilt ist, das eine kleinere Querschnittsfläche als das zweite Lumen (112) aufweist, **dadurch gekennzeichnet, dass**
das dritte Lumen (113) und das vierte Lumen (114) einander zugewandt sind, wobei die Trennwand (115) dazwischen eingefügt ist,
das zweite Lumen (112) an seinem distalen Ende eine zweite Öffnungsebene (122) aufweist und das erste Lumen (111) an seinem distalen Ende eine erste Öffnungsebene (121) aufweist, wobei die zweite Öffnungsebene (122) weiter distal liegt als die erste Öffnungsebene (121),
das dritte Lumen (113) an seinem distalen Ende eine dritte Öffnungsebene (123) aufweist, wobei die dritte Öffnungsebene (123) weiter distal liegt als die zweite Öffnungsebene (122),
das vierte Lumen (114) an seinem distalen Ende eine vierte Öffnungsebene (124) aufweist, wobei die vierte Öffnungsebene (124) weiter distal liegt als die dritte Öffnungsebene (123), und
die Trennwand (115) derart ist, dass zwischen dem distalen Ende des Körpers (101) und der zweiten Öffnungsebene (122) eine Breite der Trennwand (115) zum dritten Lumen (113) und dem vierten Lumen (114) hin allmählich schmäler wird.

2. Vierfachlumen-Katheter nach Anspruch 1, wobei
das erste Lumen (111) und das zweite Lumen (112) in Bezug auf die Trennwand (115) symmetrisch sind und das dritte Lumen (113) und das vierte Lumen (114) in Bezug auf die Trennwand (115) symmetrisch sind.

3. Vierfachlumen-Katheter nach Anspruch 1 oder 2, wobei
die erste Öffnungsebene (121) und die zweite Öffnungsebene (122) die gleiche Größe aufweisen.

4. Vierfachlumen-Katheter nach einem der Ansprüche 1 bis 3, wobei
die erste Öffnungsebene (121), die zweite Öffnungsebene (122) und die dritte Öffnungsebene (123) in Bezug auf die Trennwand (115) zum proximalen Ende des Körpers (101) hin geneigt sind, und
die vierte Öffnungsebene (124) orthogonal zur Trennwand (115) ist.

5. Vierfachlumen-Katheter nach einem der Ansprüche 1 bis 4, wobei
ein Abschnitt des Körpers (101) zwischen dem distalem Ende desselben und der zweiten Öffnungsebene (122) eine geringere Härte aufweist als ein Abschnitt des Körpers (101) zwischen der zweiten Öffnungsebene (122) und dem proximalen Ende.

6. Vierfachlumen-Katheter nach einem der Ansprüche 1 bis 5, umfassend:
eine Vielzahl von Zweigrohren (103A, 103B, 103C, 103D), die am proximalen Ende des Körpers (101) vorgesehen sind, wobei die Vielzahl von Zweigrohren (103A, 103B, 103 C, 103D) jeweils mit dem ersten Lumen (111), dem zweiten Lumen (112), dem dritten Lumen (113) und dem vierten Lumen (114) verbunden sind und jeweils mit einem Verbinder an einem proximalen Ende derselben versehen sind.

7. Vierfachlumen-Katheter nach einem der Ansprüche 1 bis 6, wobei
das erste Lumen (111) eine Vielzahl von ersten lumenseitigen Poren (125) in seinem distalen Endabschnitt aufweist, und
das zweite Lumen (112) eine Vielzahl von zweiten lumenseitigen Poren (126) in seinem distalen Endabschnitt aufweist.

8. Vierfachlumen-Katheter nach einem der Ansprüche 1 bis 7, wobei
die Außenwand (116) in einem Abschnitt, in dem das erste Lumen (111) vorgesehen ist, einen ersten Lumenschlitz (127), der ein distales Ende der Außenwand (116) einkerbt, und/oder in einem Abschnitt, in dem das zweite Lumen (112) vorgesehen ist, einen zweiten Lumenschlitz (128) aufweist, der das distale Ende einkerbt.

## Revendications

1. Cathéter à quatre lumières, comprenant :
un corps cylindrique (101) s'étendant d'une extrémité proximale à une extrémité distale et entouré d'une paroi extérieure (116),
le corps (101) ayant un espace intérieur divisé en une première région (117) et une deuxième région (118) par une cloison (115) s'étendant dans une direction longitudinale,
la première région (117) étant divisée en une première lumière (111) et une troisième lumière (113) dont la section transversale est plus petite que celle de la première lumière (111),
la deuxième région (118) étant divisée en une deuxième lumière (112) et une quatrième lumière (114) dont la section transversale est plus petite que celle de la deuxième lumière (112), **caractérisé en ce que**
la troisième lumière (113) et la quatrième lumière (114) se font face avec la cloison (115) interposée entre elles,
la deuxième lumière (112) a un deuxième plan d'ouverture (122) au niveau de son extrémité distale, et la première lumière (111) a un premier plan d'ouverture (121) au niveau de son extrémité distale, le deuxième plan d'ouverture (122) étant plus distal que le premier plan d'ouverture (121),
la troisième lumière (113) a un troisième plan d'ouverture (123) au niveau de son extrémité distale, le troisième plan d'ouverture (123) étant plus distal que le deuxième plan d'ouverture (122),
la quatrième lumière (114) a un quatrième plan d'ouverture (124) au niveau de son extrémité distale, le quatrième plan d'ouverture (124) étant plus distal que le troisième plan d'ouverture (123), et
la cloison (115) est telle que, entre l'extrémité distale du corps (101) et le deuxième plan d'ouverture (122), une largeur de la cloison (115) se rétrécit progressivement vers la troisième lumière (113) et la quatrième lumière (114).

2. Cathéter à quatre lumières de la revendication 1, dans lequel
la première lumière (111) et la deuxième lumière (112) sont symétriques par rapport à la cloison (115), et la troisième lumière (113) et la quatrième lumière (114) sont symétriques par rapport à la cloison (115).

3. Cathéter à quatre lumières de la revendication 1 ou 2, dans lequel
le premier plan d'ouverture (121) et le deuxième plan d'ouverture (122) sont de taille identique.

4. Cathéter à quatre lumières de l'une quelconque des revendications 1 à 3, dans lequel
le premier plan d'ouverture (121), le deuxième plan d'ouverture (122) et le troisième plan d'ouverture (123) sont inclinés vers l'extrémité proximale du corps (101) par rapport à la cloison (115), et
le quatrième plan d'ouverture (124) est orthogonal à la cloison (115).

5. Cathéter à quatre lumières de l'une quelconque des revendications 1 à 4, dans lequel
une partie du corps (101) entre son extrémité distale et le deuxième plan d'ouverture (122) présente une dureté inférieure à celle d'une partie du corps (101) entre le deuxième plan d'ouverture (122) et l'extrémité proximale.

6. Cathéter à quatre lumières de l'une quelconque des revendications 1 à 5, comprenant :
une pluralité de tuyaux de dérivation (103A, 103B, 103C, 103D) prévus à l'extrémité proximale du corps (101), la pluralité de tuyaux de dérivation (103A, 103B, 103C, 103D) étant reliés respectivement à la première lumière (111), à la deuxième lumière (112), à la troisième lumière (113) et à la quatrième lumière (114) et étant chacun pourvu d'un raccord au niveau d'une extrémité proximale de celui-ci.

7. Cathéter à quatre lumières de l'une quelconque des revendications 1 à 6, dans lequel
la première lumière (111) a une pluralité de pores latéraux de première lumière (125) dans sa partie d'extrémité distale, et
la deuxième lumière (112) a une pluralité de pores latéraux de deuxième lumière (126) dans sa partie d'extrémité distale.

8. Cathéter à quatre lumières de l'une quelconque des revendications 1 à 7, dans lequel
la paroi extérieure (116) a, dans une partie où la première lumière (111) est prévue, une première fente de lumière (127) entaillant une extrémité distale de la paroi extérieure (116) et/ou dans une partie où la deuxième lumière (112) est prévue, une deuxième fente de lumière (128) entaillant l'extrémité distale.
